# EUROPEAN PATENT APPLICATION

(11) **EP 4 516 297 A1**
(43) Date of publication of application: **05.03.2025**
(21) Application number: 22939292.3
(22) Date of filing: 29.04.2022
(51) Int. Cl.: A61K 9/72, A61K 9/08, A61K 31/5578, A61K 47/18, A61K 47/02, A61K 47/26, A61P 11/00, A61P 9/12

(54) **TREPROSTINIL SOFT MIST INHALANT**

(71) Applicant: Zhaoke Pharmaceutical (Guangzhou) Co., Ltd, Guangzhou, Guangdong 511466 (CN)
(72) Inventor: DAI, Xiangrong, Guangzhou, Guangdong 511466 (CN); LI, Xiaoyi, Guangzhou, Guangdong 511466 (CN); YIN, Lei, Guangzhou, Guangdong 511466 (CN); CHEN, Linqing, Guangzhou, Guangdong 511466 (CN)
(74) Representative: Isern Patentes y Marcas S.L.
(86) International application number: PCT/CN2022/090503
(87) International publication number: WO 2023/206444

(57) **Abstract**

The present disclosure provides a liquid propellant-free pharmaceutical preparation including treprostinil. The pharmaceutical preparation can undergo a better nebulization effect in a soft mist inhaler than the existing treprostinil preparations. In addition, the active substance in the pharmaceutical preparation is stable during a storage period of 1 to 2 years or longer.

## Description

### TECHNICAL FIELD

The present disclosure belongs to the field of pharmaceutical preparations, and in particular relates to a treprostinil inhalable preparation.

### BACKGROUND TECHNOLOGY

Treprostinil, [[(1R,2R,3aS,9aS)-2,3,3a,4,9,9a-hexahydro-2-hydroxy-1-[(3S)-3-hydroxyoctyl]-1H-benz[f]inden-5-yl]oxy]acetic acid, has the following chemical formula:

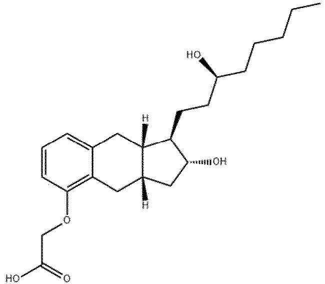

Currently, there are the following three preparations of treprostinil on the market: subcutaneous or intravenous injections (Remodulin, 20 mL : 20 mg), extended-release tablets (Orenitram, 0.125 m/0.25 mg), and inhalants nebulized by ultrasonic pulses (Tyvaso, 1.74 mg/0.29 mL).

According to "the voice of the patient," it is proposed in a patient-focused drug development initiative issued by the Food and Drug Administration (FDA) that patients are seeking a convenient and effective treatment to improve a series of symptoms affecting daily life caused by pulmonary hypertension. An administration mode that can be easily implemented and can be applied anytime and anywhere is preferred instead of an administration mode that requires an immediate halt of any ongoing activities (including working, going to the toilet, bathing, etc.).

The inhalation administration refers to an administration mode in which one or more drugs are delivered to the lungs deep in the respiratory tract through a special delivery device to exert a local or systemic effect. Inhalable preparations mainly include the following three categories: inhalation aerosols, inhalation powder aerosols, and liquid preparations for nebulizers.

Tyvaso is a liquid preparation for nebulizers and is a treprostinil inhalation system (see U.S. patent US10076505) developed by the United Therapeutics Corporation. This liquid pharmaceutical preparation of treprostinil can be converted into a mist form by a nebulizer and then inhaled into the lungs of the human body to achieve a drug medication effect. However, because pulmonary hypertension is a chronic disease and is prevalent in people aged 20 to 40 generally, patients with moderate to mild pulmonary hypertension who are undergoing treatments can still go out and work normally. Due to product requirements, the aerosol inhalant Tyvaso requires the use of a specific nebulizer that is bulky and not easy to carry. When the nebulizer with the liquid preparation is used, a patient needs to take the pharmaceutical liquid four times a day when awake at an interval of 4 h; an external power supply may be required, and the nebulizer should not be placed flat or dumped during use or standby. Therefore, the daily use of the aerosol inhalant Tyvaso for patients going out is still not convenient and affects the quality of work and life.

According to the U.S. patent US10898494, Liquid Technologies developed a treprostinil dry powder inhalant, which is an inhalation powder aerosol. In this patent, 25 µg of treprostinil is prepared into a dry granular preparation and encapsulated in a capsule, and then can be inhaled into a patient through a dry powder inhaler. However, drug particles in the dry powder inhalant and the inhalation powder aerosol have a small particle size (1 µm to 5 µm). The agglomeration among the drug particles is enhanced due to strong interactions among the drug particles (van der Waals forces, electrostatic forces, and capillary forces), such that, during storage, the drug particles easily agglomerate to make the particle size change and affect the efficacy. Therefore, carrier particles are usually required to hinder the agglomeration of a drug powder. Generally, carrier particles having a particle size of 70 µm to 100 µm are mixed with a drug powder having a particle size of 0.5 µm to 7 µm, such that the drug powder is adsorbed on the surface of the carrier particles. During the inhalation by a patient, the drug powder is separated from the carrier particles, enters the lungs of the human body, and is deposited. For example, the U.S. patent 10076505 discloses a formula of 1.06% of treprostinil sodium, 92.44% of trehalose dihydrate, 2% of polysorbate 80, 4% of L-leucine, 0.27% of sodium citrate dihydrate, and 0.23% of sodium chloride. In this patent, a treprostinil sodium powder is mixed with the macromolecular polysaccharide compound trehalose having a large particle size as a carrier to prepare a treprostinil inhalant. However, when the treprostinil inhalant is inhaled by a patient, only about 20% to 30% of the drug is delivered to the lungs, and a large amount of the drug and the adjuvant is deposited in the mouth and throat and finally enters the stomach, which may cause adverse side effects through the digestive system. In addition, the treprostinil inhalant can easily cause a strong foreign body sensation, and for patients with pharyngeal sensitivity, the treprostinil inhalant is more likely to cause irritable cough, which affects the inhalation experience.

The U.S. patent US20190030268 discloses a soft mist inhalation device in detail, which is a propellant-free liquid drug inhalation device for metered administration. A pharmaceutical preparation solution in a nebulizer is converted into an aerosol acting on the lungs, and the pharmaceutical preparation solution is sprayed out by the nebulizer using the same high-pressure method as in various countries. This inhalation device is particularly suitable for the liquid preparation of the present disclosure. It has a length of about 8 cm to about 18 cm and a width of about 2.5 cm to about 5 cm, is small, and can be easily operated. The inhalation device can be taken anywhere by patients, which allows great convenience for the medication of patients.

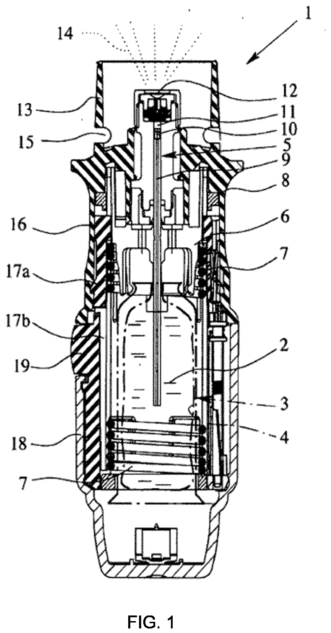

FIG. 1 shows a cross section of the inhalation device under pressure. The inhalation device includes a sprayer 1, a liquid 2, a container 3, a liquid compartment 4, a pressure generator 5, a support 6, a driving spring 7, a conveying pipe 9, a check valve 10, a spray nozzle 12, a suction nozzle 13, an aerosol 14, an air inlet 15, an upper shell 16, an internal component 17, and a lower shell 18.

The inhalable solution of the present disclosure can be administered with the inhalation device above. A small amount of the inhalable solution, about less than 70 µL, such as less than about 30 µL or less than about 15 µL of the inhalable solution, can be nebulized in a single spray, such that an inhalable part of the aerosol corresponds to a therapeutically effective amount. The aerosol sprayed has an average particle size of less than 10 µm or 15 µm. The detailed description of the soft mist inhalation device can be seen in the U.S. patent US20190030268.

The soft mist inhalation device disclosed in the U.S. patent US20190030268 has a length of about 8 cm to about 18 cm and a width of about 2.5 cm to about 5 cm, is small, and can be easily operated. This soft mist inhalation device can be taken anywhere by patients, which allows great convenience for the medication of patients.

However, there has been no soft mist inhalant for treprostinil administration on the market. Compared with the Tyvaso aerosol inhalation device on the market, the soft mist inhalation device is small and convenient, has lower requirements on the inspiratory flow rate of the patient, and can extend an administration time and a spray duration. As a result, the patient only needs to breathe naturally to make a drug fully deposited in the lungs, which can improve the availability. Compared with the dry powder inhalants or the inhalation powder aerosols, the soft mist inhalation device can also improve the foreign body sensation in the throat and reduce the administration dose.

### CONTENT OF THE INVENTION

The present disclosure provides a propellant-free liquid pharmaceutical preparation and a method for administering the pharmaceutical preparation by nebulizing the pharmaceutical preparation in a soft mist inhaler. The pharmaceutical preparation can meet the desired criteria to undergo the optimal nebulization in the soft mist inhaler. The present disclosure can ensure that an active substance in the pharmaceutical preparation is stable during a storage period of several years (which may specifically be 1 or 2 years).

A first aspect of the present disclosure provides a pharmaceutical preparation, including: (a) treprostinil and/or a pharmaceutically-acceptable salt of the treprostinil; (b) a solvent; (c) a pharmaceutically-acceptable solubilizer; and (d) a pharmaceutically-acceptable preservative.

Further, the pharmaceutical preparation is in a liquid state.

Further, the pharmaceutical preparation does not include a propellant.

Further, the treprostinil or the pharmaceutically-acceptable salt of the treprostinil is one or more of respective stereoisomers of the treprostinil or the pharmaceutically-acceptable salt of the treprostinil.

Further, the treprostinil is one or a mixture of two or more of various crystal forms of treprostinil, such as a crystal form mentioned in US9822057B2, a crystal form mentioned in EP2970091A1, or various forms of treprostinil disclosed in the following U.S. patents: U.S. patent application Nos. 5153222, 5234953, 6521212, 6756033, 6803386, 7199157, 6054486, 7417070, 7384978, 7879909, 8563614, 8252839, 8536363, 8410169, 8232316, 8609728, 8350079, 8349892, 7999007, 8658694, 8653137, 9029607, 8765813, 9050311, 9199908, 9278901, 8747897, 9358240, 9339507, 9255064, 9278902, 9278903, 9758465, 9422223, 9878972, and 9624156; U.S. patent application Nos. 2009-0036465, 2008-0200449, 2008-0280986, 2008-024697, 2010-0275616, 2010-0275616, 2011-0184262, 2011-0184295, 2011-0323567, 2011-0323567, 2010323567, 20103-0323567, 2016-0030356, 2016-0030356, 2016-0051505, 2016-0051505, 2016-0030355, 2016-0143868, 2010-0328232, 2015-0148414, 2016-0045470, 2016-0129087, 2010-0129087, 2010-0095432, and 2018-0153847; and patents with PCT publication Nos. WO00/57701, WO2016010538, WO2016038532, WO2016038532, and WO2018/058124.

In some specific embodiments, the pharmaceutically-acceptable salt of the treprostinil is one or more of salts of treprostinil involved in the patent US9988334B2.

In some specific embodiments, the pharmaceutically-acceptable salt of the treprostinil is selected from one of a sodium salt, a calcium salt, a diethanolamine salt, a triethanolamine salt, an L-arginine salt, a lysine salt, a meglumine salt, an ammonium salt, and a choline salt of the treprostinil.

Further, a content of the treprostinil or the pharmaceutically-acceptable salt thereof in the pharmaceutical preparation is in a range of about 0.086 mg/mL to about 0.6 mg/mL. In some specific embodiments, the content is in a range of 0.080 mg/mL to 0.12 mg/mL. In some specific embodiments, the content is in a range of 0.13 mg/mL to 0.18 mg/mL. In some specific embodiments, the content is in a range of 0.19 mg/mL to 0.3 mg/mL. In some specific embodiments, the content is in a range of 0.4 mg/mL to 0.6 mg/mL.

Further, the solvent is selected from an organic solvent or a mixed solvent of water and an organic solvent. Preferably, the organic solvent is selected from one or more of an alcohol, N-methylpyrrolidone, benzyl benzoate, and dimethyl sulfoxide. More preferably, the alcohol is selected from methanol, ethanol, n-propanol, isopropanol, n-butanol, isobutanol, tert-butanol, ethylene glycol, propylene glycol, glycerin, benzyl alcohol, phenethyl alcohol, and polyethylene glycol. In particular, preferably, the solvent is selected from a combination of ethanol and benzyl alcohol, or a combination of ethanol, benzyl alcohol, and water, or a combination of ethanol, benzyl alcohol, and N-methylpyrrolidone, or a combination of ethanol, benzyl alcohol, and dimethyl sulfoxide.

In some specific embodiments, the solvent is a mixture of water and ethanol, and the ethanol has a specified permeation-promoting effect. In a specific embodiment, an amount of ethanol is about 100 mg/mL to about 300 mg/mL. In some specific embodiments, the amount of ethanol is 100 mg/mL to 150 mg/mL. In some specific embodiments, the amount of ethanol is 200 mg/mL to 300 mg/mL. In some specific embodiments, the amount of ethanol is 160 mg/mL to 200 mg/mL. In some specific embodiments, the amount of ethanol is 210 mg/mL to 250 mg/mL. In some specific embodiments, the amount of ethanol is 250 mg/mL to 300 mg/ mL.

Further, in some specific embodiments, a stabilizer in the pharmaceutical preparation is tromethamine, and the tromethamine also serves as an alkaline regulator or a buffer. Preferably, an amount of the tromethamine is about 0.05 mg/mL to about 0.2 mg/mL. In other specific embodiments, the stabilizer is sodium hydroxide, sodium citrate, or Tween-80.

In some embodiments, the pharmaceutical preparation may further include one or more osmotic pressure regulators. The one or more osmotic pressure regulators are selected from one or more of sodium chloride, magnesium chloride, glucose, sodium chloride, a phosphate, and a citrate. In some specific embodiments, when adopted as an osmotic pressure regulator, sodium chloride can adjust an osmotic pressure of the whole solution to an optimal value of 300±30 mOsm/kg. In a specific embodiment, an amount of the overall sodium chloride is about 0.1 mg/mL to about 9 mg/mL, such as 0.1 mg/mL, 0.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, and 9.0 mg/mL.

Further, the pharmaceutical preparation may further include one or more preservatives that can provide an antimicrobial activity and may further provide a stability benefit. Preferably, the one or more preservatives are aromatic acids and phenol compounds or mixtures of these compounds. In some specific embodiments, the one or more preservatives each are selected from, but are not limited to, one or more of Tween 80, sodium citrate, m-cresol, sodium benzoate, chlorhexidine acetate, chlorhexidine gluconate, and 50% benzalkonium chloride. In some specific embodiments, the one or more preservatives are 50% benzalkonium chloride, which can also serve as a surfactant and a solubilizer. Preferably, an amount of 50% benzalkonium chloride is about 0.1 mg/mL to about 9 mg/mL, such as 0.1 mg/mL, 0.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, and 9.0 mg/mL.

In some embodiments, the pharmaceutical preparation further includes an acidity regulator. In a specific embodiment, the acidity regulator is hydrochloric acid. In some other specific embodiments, the acidity regulator may also be selected from, but are not limited to, other pharmaceutically-acceptable acidity regulators such as citric acid, benzoic acid, and acetic acid. Preferably, a pH of the pharmaceutical preparation is 6.5 to 8.5.

In some specific embodiments, the pharmaceutical preparation is an inhalable preparation.

In some other specific embodiments, the pharmaceutical preparation is an inhalable preparation, which may be nebulized by a soft mist inhalation device or a compressed air nebulizer for administration. Preferably, the pharmaceutical preparation is administered with a soft mist inhalation device, and more preferably, the pharmaceutical preparation is administered with the soft mist inhalation device disclosed in the U.S. patent US20190030268.

Further, the pharmaceutical preparation has a low requirement for an inspiratory flow rate of a patient, and a patient only needs to breathe naturally to make a drug fully deposited in lungs. Then, the delivery of an inhaled drug is improved by increasing the deposition in lungs.

The propellant-free liquid pharmaceutical preparation including treprostinil or a pharmaceutically-acceptable salt thereof provided in the present disclosure can undergo the optimal nebulization in a soft mist inhaler. The present disclosure can ensure that the active substance in the pharmaceutical preparation is stable during a storage period of several years (which may specifically be 1 or 2 years).

### DESCRIPTION OF THE DRAWINGS

FIG. 1 is a cross-sectional view of a soft mist inhalation device under pressure; and
FIG. 2 shows the chemical stability of a main component within 24 months.

### SPECIFIC IMPLEMENTATIONS

The present disclosure is intended to provide a propellant-free liquid pharmaceutical preparation including treprostinil, and the pharmaceutical preparation can meet the desired criteria to undergo the optimal nebulization in a soft mist inhaler.

The "soft mist inhaler" below refers to the propellant-free liquid drug inhalation device for metered administration disclosed in the U.S. patent US20190030268. This propellant-free liquid drug inhalation device is particularly suitable for the inhalable preparation of the present disclosure. An aerosol produced through a spray should have a particle size of less than 10 µm and preferably less than 5 µm.

The treprostinil used below comes from the Chirogate International Inc., and has a Batch No. L-200-727.

An inhalation device adopted for Tyvaso is Nebu-tec Optineb-irModelON-100/7, which is an ultrasonic vibrating mesh nebulizer with models TD-100 and TD-300.

The marketed Tyvaso inhalable preparation needs to be administered four times a day, with 3 breaths each time and a dose of about 6 µg each breath. According to this effective clinical data, a solvent content for the treprostinil inhalable preparation of the present disclosure is formulated.

The inhalation device mentioned in the U.S. patent US20190030268 can nebulize a small amount of a liquid in a single spray, about less than 70 µL, such as less than about 30 µL or less than about 15 µL of a drug solution. Thus, the treprostinil inhalable preparation of the present disclosure should include about 0.086 mg/mL to about 0.4 mg/mL of treprostinil.

### Example 1 Preparation of inhalable solutions of sample 1 and sample 2

The corresponding components were listed in Table 1. According to Table 1: Treprostinil and 50% benzalkonium chloride were placed in a 100 mL volumetric flask, and 95% ethanol was added for dissolution. Tromethamine and sodium chloride were added, an appropriate amount of water for injection was added, and an ultrasonic treatment was conducted for complete dissolution to obtain a mixed solution. Finally, the mixed solution was diluted with purified water to a specified volume and then adjusted to a corresponding pH.

**Table 3 Component contents of 100 mL inhalable solutions for the samples 1 and 2**

| Component | Sample 1 | Sample 2 |
|---|---|---|
| Treprostinil | 8.6 mg | 60 mg |
| Tromethamine | 10 mg | 10 mg |
| Sodium chloride | 500 mg | 500 mg |
| 50% benzalkonium chloride | 20 mg | 20 mg |
| 95% ethanol | 20 g | 20 g |
| Water for injection | Adding to 100 mL | Adding to 100 mL |
| Adjusting a pH with HCl to | 6.8 | 6.8 |

**Table 2 Content determination of the inhalable solutions for the samples 1 and 2**

| Sample No. | Component | Concentration (mg/mL) | Content determination (%) |
|---|---|---|---|
| Sample 1 | Treprostinil | 0.0879 | 98.81 |
| Sample 2 | Treprostinil | 0.5972 | 100.20 |

### Example 2 Preparation of inhalable solutions of sample 3, sample 4, and

### sample 5

The corresponding components were listed in Table 3. According to Table 3: Treprostinil and 50% benzalkonium chloride were placed in a 100 mL volumetric flask, and 95% ethanol was added for dissolution. Tromethamine and sodium chloride were added, an appropriate amount of water for injection was added, and an ultrasonic treatment was conducted for complete dissolution to obtain a mixed solution. Finally, the mixed solution was diluted with purified water to a specified volume and then adjusted to a corresponding pH.

**Table 3 Component contents of 100 mL inhalable solutions for the samples 3, 4, and 5**

| Component | Sample 3 | Sample 4 | Sample 5 |
|---|---|---|---|
| Treprostinil | 30 mg | 30 mg | 30 mg |
| Tromethamine | 10 mg | 10 mg | 10 mg |
| Sodium chloride | 500 mg | 500 mg | 500 mg |
| 50% benzalkonium chloride | 5 mg | 20 mg | 30 mg |
| 95% ethanol | 20 g | 20 g | 20 g |
| Water for injection | Adding to 100 mL | Adding to 100 mL | Adding to 100 mL |
| Adjusting a pH with HCl to | 6.8 | 6.8 | 6.8 |

**Table 4 Content determination of the inhalable solutions for the samples 3, 4, and 5**

| Sample No. | Component | Concentration (mg/mL) | Content determination (%) |
|---|---|---|---|
| Sample 3 | Treprostinil | 0.2977 | 98.02 |
| Sample 4 | Treprostinil | 0.3062 | 97.47 |
| Sample 5 | Treprostinil | 0.2908 | 99.69 |

### Example 3 Preparation of inhalable solutions of sample 6 and sample 7

The corresponding components were listed in Table 5. According to Table 5: Treprostinil and 50% benzalkonium chloride were placed in a 100 mL volumetric flask, and 95% ethanol was added for dissolution. Tromethamine and sodium chloride were added, an appropriate amount of water for injection was added, and an ultrasonic treatment was conducted for complete dissolution to obtain a mixed solution. Finally, the mixed solution was diluted with purified water to a specified volume and then adjusted to a corresponding pH.

**Table 5 Component contents of 100 mL inhalable solutions for the samples 6 and 7**

| Component | Sample 6 | Sample 7 |
|---|---|---|
| Treprostinil | 30 mg | 30 mg |
| Sodium chloride | 500 mg | 500 mg |
| Tromethamine | 5 mg | 20 mg |
| 50% benzalkonium chloride | 20 mg | 20 mg |
| 95% ethanol | 20 g | 20 g |
| Water for injection | Adding to 100 mL | Adding to 100 mL |
| Adjusting a pH with HCl to | 6.8 | 6.8 |

**Table 6 Content determination of the inhalable solutions for the samples 6 and 7**

| Sample No. | Component | Concentration (mg/mL) | Content determination (%) |
|---|---|---|---|
| Sample 6 | Treprostinil | 0.3029 | 99.56 |
| Sample 7 | Treprostinil | 0.3081 | 99.70 |

### Example 4 Preparation of inhalable solutions of sample 8 and sample 9

The corresponding components were listed in Table 7. According to Table 7: Treprostinil and 50% benzalkonium chloride were placed in a 100 mL volumetric flask, and 95% ethanol was added for dissolution. Tromethamine and sodium chloride were added, an appropriate amount of water for injection was added, and an ultrasonic treatment was conducted for complete dissolution to obtain a mixed solution. Finally, the mixed solution was diluted with purified water to a specified volume and then adjusted to a corresponding pH.

**Table 7 Component contents of 100 mL inhalable solutions for the samples 8 and 9**

| Component | Sample 8 | Sample 9 |
|---|---|---|
| Treprostinil | 30 mg | 30 mg |
| Sodium chloride | 10 mg | 900 mg |
| Tromethamine | 10 mg | 10 mg |
| 50% benzalkonium chloride | 20 mg | 20 mg |
| 95% ethanol | 20 g | 20 g |
| Water for injection | Adding to 100 mL | Adding to 100 mL |
| Adjusting a pH with HCl to | 6.8 | 6.8 |

**Table 8 Content determination of the inhalable solutions for the samples 8 and 9**

| Sample No. | Component | Concentration (mg/mL) | Content determination (%) |
|---|---|---|---|
| Sample 8 | Treprostinil | 0.2977 | 98.24 |
| Sample 9 | Treprostinil | 0.3102 | 97.35 |

### Example 5 Preparation of inhalable solutions of sample 10 and sample 11

The corresponding components were listed in Table 9. According to Table 9: Treprostinil and 50% benzalkonium chloride were placed in a 100 mL volumetric flask, and 95% ethanol was added for dissolution. Tromethamine and sodium chloride were added, an appropriate amount of water for injection was added, and an ultrasonic treatment was conducted for complete dissolution to obtain a mixed solution. Finally, the mixed solution was diluted with purified water to a specified volume and then adjusted to a corresponding pH.

**Table 9 Component contents of 100 mL inhalable solutions for the samples 10 and 11**

| Component | Sample 10 | Sample 11 |
|---|---|---|
| Treprostinil | 30 mg | 30 mg |
| Sodium chloride | 500 mg | 500 mg |
| Tromethamine | 10 mg | 10 mg |
| 50% benzalkonium chloride | 20 mg | 20 mg |
| 95% ethanol | 10 g | 30 g |
| Water for injection | Adding to 100 mL | Adding to 100 mL |
| Adjusting a pH with HCl to | 6.8 | 6.8 |

**Table 10 Content determination of the inhalable solutions for the samples 10 and 11**

| Sample No. | Component | Concentration (mg/mL) | Content determination (%) |
|---|---|---|---|
| Sample 10 | Treprostinil | 0.2977 | 99.02 |
| Sample 11 | Treprostinil | 0.3102 | 100.15 |

### Example 6 Preparation of inhalable solutions of sample 12 and sample 13

The corresponding components were listed in Table 11. According to Table 9: Treprostinil and 50% benzalkonium chloride were placed in a 100 mL volumetric flask, and 95% ethanol was added for dissolution. Tromethamine and sodium chloride were added, an appropriate amount of water for injection was added, and an ultrasonic treatment was conducted for complete dissolution to obtain a mixed solution. Finally, the mixed solution was diluted with purified water to a specified volume and then adjusted to a corresponding pH.

**Table 11 Component contents of 100 mL inhalable solutions for the samples 12 and 13**

| Component | Sample 12 | Sample 13 |
|---|---|---|
| Treprostinil | 30 mg | 30 mg |
| Sodium chloride | 500 mg | 500 mg |
| Tromethamine | 10 mg | 10 mg |
| 50% benzalkonium chloride | 15 mg | 15 mg |
| 95% ethanol | 20 g | 20 g |
| Water for injection | Adding to 100 mL | Adding to 100 mL |
| Adjusting a pH with HCl to | 6.5 | 8.5 |

**Table 12 Content determination of the inhalable solutions for the samples 12 and 13**

| Sample No. | Component | Concentration (mg/mL) | Content determination (%) |
|---|---|---|---|
| Sample 12 | Treprostinil | 0.3046 | 97.13 |
| Sample 13 | Treprostinil | 0.2977 | 99.22 |

### Example 7

The samples 3, 4, and 5 each were nebulized with a soft mist inhaler, a compressed air nebulizer (PARI SINUS), and an ultrasonic vibrating mesh nebulizer (TD-7001). A particle size distribution of droplets sprayed was then measured with Sprattec (STP5311, Malvern). Test results are shown in Table 13. D50 values of the samples 3, 4, and 5 all are less than 10 µm, but the soft mist inhaler can produce a small and uniform particle size distribution change.

**Table 13 Determination of nebulized particle sizes of the samples 3, 4, and 5**

| Sample No. | Particle size | Nebulization with the soft mist inhalation device | Nebulization with the compressed air nebulizer | Nebulization with the ultrasonic vibrating mesh nebulizer |
|---|---|---|---|---|
| Sample 3 | D10 | 2.57 | 1.624 | 2.397 |
| | D50 | 4.507 | 5.441 | 4.133 |
| | D90 | 8.951 | 10.77 | 9.587 |
| Sample 4 | D10 | 2.531 | 1.817 | 2.003 |
| | D50 | 4.609 | 5.176 | 4.027 |
| | D90 | 8.84 | 11.02 | 8.61 |
| Sample 5 | D10 | 2.327 | 1.752 | 2.11 |
| | D50 | 4.248 | 5.274 | 4.407 |
| | D90 | 8.611 | 11.2 | 9.962 |

### Example 8 Testing of chemical stability of an active ingredient in each of the pharmaceutical preparations in Examples 1 to 7

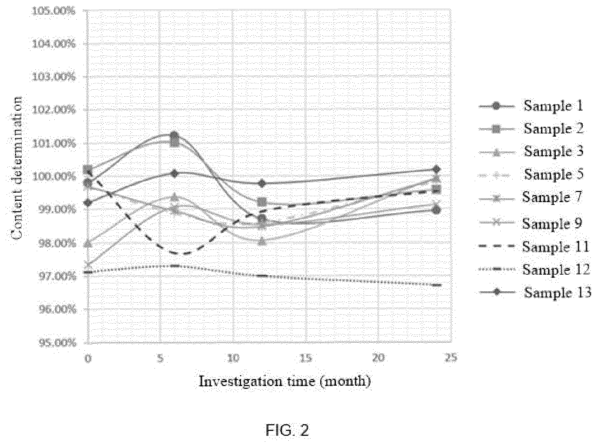

The samples 1, 2, 3, 5, 7, 9, 11, 12, and 13 in Examples 1 to 7 each were placed at 15°C to 25°C for 6 months, 12 months, and 24 months, and then a content of the active ingredient was determined. Test results are shown in Table 14, and the trend comparison is shown in FIG. 2.

**Table 14 Stability investigation results of Examples 1 to 7**

| Sample | Content determination | | | |
|---|---|---|---|---|
| | Month 0 | Month 6 | Month 12 | Month 24 |
| Sample 1 | 99.81% | 101.23% | 98.71% | 98.96% |
| Sample 2 | 100.20% | 101.01% | 99.21% | 99.58% |
| Sample 3 | 98.02% | 99.37% | 98.06% | 99.94% |
| Sample 5 | 99.69% | 98.89% | 98.60% | 99.82% |
| Sample 7 | 99.70% | 98.96% | 98.49% | 99.90% |
| Sample 9 | 97.35% | 99.07% | 98.53% | 99.13% |
| Sample 11 | 100.15% | 97.68% | 98.92% | 99.53% |
| Sample 12 | 97.13% | 97.30% | 96.99% | 96.71% |
| Sample 13 | 99.22% | 100.09% | 99.77% | 100.18% |

The above are merely preferred examples of the present disclosure and are not intended to limit the present disclosure, and various changes and modifications can be made by those skilled in the art to the present disclosure. Any modifications, equivalent substitutions, improvements, etc. made within the spirit and scope of the present disclosure should be included within the protection scope of the present disclosure.

## Claims

1. A propellant-free liquid pharmaceutical preparation, comprising: (a) treprostinil or a pharmaceutically-acceptable salt of the treprostinil; (b) a solvent; (c) a pharmaceutically-acceptable solubilizer; and (d) a pharmaceutically-acceptable preservative.

2. The pharmaceutical preparation according to claim 1, wherein a content of the treprostinil or the pharmaceutically-acceptable salt of the treprostinil is in a range of 0.086 mg/mL to 0.6 mg/mL.

3. The pharmaceutical preparation according to claim 1, wherein the solvent is a mixture of water and ethanol, and a content of the ethanol is 100 mg/mL to 300 mg/mL.

4. The pharmaceutical preparation according to claim 1, wherein the solubilizer is tromethamine, and a content of the tromethamine is 0.05 mg/mL to 0.2 mg/mL.

5. The pharmaceutical preparation according to claim 1, further comprising an osmotic pressure regulator, wherein an osmotic pressure of the pharmaceutical preparation is 300±30 mOsm/kg.

6. The pharmaceutical preparation according to claim 5, wherein the osmotic pressure regulator is selected from one or more of glucose, sodium chloride, magnesium chloride, a phosphate, or a citrate.

7. The pharmaceutical preparation according to claim 1, wherein the preservative is a combination of one or more of Tween 80, sodium citrate, m-cresol, sodium benzoate, chlorhexidine acetate, chlorhexidine gluconate, and 50% benzalkonium chloride.

8. The pharmaceutical preparation according to claim 1, further comprising an acidity regulator, wherein a pH of the pharmaceutical preparation is 6.5 to 8.5.

9. The pharmaceutical preparation according to claim 8, wherein the acidity regulator is selected from one or more of hydrochloric acid, citric acid, benzoic acid, and acetic acid.

10. The pharmaceutical preparation according to any one of claims 1 to 9, wherein the pharmaceutical preparation is an inhalable preparation that is able to be nebulized by a soft mist inhalation device or a compressed air nebulizer for an administration.
